(19) Europäisches Patentamt
European Patent Office
Office européen des brevets

(11) **EP 3 518 852 B1**

(12) **EUROPEAN PATENT SPECIFICATION**

(45) Date of publication and mention
of the grant of the patent:
**30.09.2020 Bulletin 2020/40**

(51) Int Cl.:
***A61F 13/537*** *(2006.01)*      ***B32B 5/26*** *(2006.01)*
***D04H 3/018*** *(2012.01)*      ***D04H 3/147*** *(2012.01)*

(21) Application number: **17797851.7**

(22) Date of filing: **29.09.2017**

(86) International application number:
**PCT/CZ2017/050045**

(87) International publication number:
**WO 2018/059610 (05.04.2018 Gazette 2018/14)**

(54) **SPUNBOND NONWOVEN WEB FOR AN ACQUISITION/DISTRIBUTION LAYER**

SPINNVLIESBAHN FÜR EINE AUFNAHME-/VERTEILUNGSSCHICHT

BANDE NON-TISSÉE FILÉE-LIÉE POUR UNE COUCHE D'ACQUISITION/DISTRIBUTION

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **30.09.2016 CZ 20160612**

(43) Date of publication of application:
**07.08.2019 Bulletin 2019/32**

(73) Proprietor: **PFNonwovens Czech s.r.o.
669 04 Znojmo (CZ)**

(72) Inventors:
• **MECL, Zdenek
 67181 Novy Saldorf - Sedlesovice (CZ)**
• **KLASKA, Frantisek
 68401 Slavkov u Brna (CZ)**
• **KROUTILOVA, Jana
 67164 Bozice (CZ)**

(74) Representative: **Zemanová, Veronika
Kania, Sedlak, Smola
Patent Attorneys
Mendlovo namesti 1 a
603 00 Brno (CZ)**

(56) References cited:
**EP-A1- 2 491 910      KR-A- 20160 083 509
US-A1- 2005 245 158      US-A1- 2015 173 975
US-B1- 6 608 236**

**Description**

FIELD OF THE INVENTION

The present invention relates to a spunbond nonwoven web for an acquisition/distribution layer, the nonwoven web comprising a first filament layer and a second filament layer, which differs from the first layer. The invention also relates to the application of such nonwoven in the absorbent hygiene products.

BACKGROUND OF THE INVENTION

Disposable absorbent articles, such as diapers, pads, sanitary towels etc., are manufactured as layered products comprising a liquid pervious topsheet, a liquid impervious backsheet joined to said topsheet; an absorbent core positioned between said topsheet and said backsheet, and an acquisition/distribution layer positioned between said topsheet and said absorbent core.

Substantially, the topsheet is expected to have excellent tactile properties and to provide soft feeling, while it allows any fluid to get quickly into the absorbent article.

The acquisition/distribution layer (also called wicking/surge layer or ADL) is sandwiched between the topsheet and the core. The ADL should have a high void volume in order to achieve satisfactory strikethrough and rewet characteristics, and sufficient temporary liquid storage capacity of ADL which in-use serves as a temporary fluid storage before a slower absorbing core empties the ADL making it prepared for the next insult. For ADL, there is a need to increase the rate with which a liquid enters the absorbent core and to decrease the rate of rewetting or to completely eliminate rewetting of the topsheet, and thus skin of wearer. This is especially problematic, when not only the first insult (the first amount of liquid) is to be absorbed by the article, but also a second or third insult, which in practical use of absorbent products often takes place especially during the night.

A further aim of current producers of disposable absorbent articles is to lower manufacturing cost and the price, which may be especially achieved by lowering of material costs, in this case the basis weight of the used nonwovens.

Therefore there is a need for a nonwoven web for an acquisition/distribution layer which has a relatively low basis weight, while it is bulky, provides a high rate with which a liquid gets through the ADL towards the absorbent core, while the rate of rewetting remains low.

US20150148764 by Latimer et al. discloses a unitary fabric structure for use within a personal care absorbent article, which includes a composite of at least two functional components for fluid intake, wherein the two functional components include a fibrous, liner functional component (topsheet) and at least one fibrous, surge functional component (ADL). The ADL includes a first component and a second component, said first component including a mixture of relatively large diameter wettable fibers from between about 25 to 40 microns, and relatively small diameter wettable fibers from between about 8 to 18 microns, said second component including relatively small diameter wettable fiber, of between about 8 to 18 microns. However, such nonwoven web does not provide satisfactory bulk per basis weight ratio and the machinery for its production is complicated (i.e. expensive), because of the necessity to produce a homogenous mixture of different fibers as the first layer. Due to the complicated structure of machinery also the operation and maintenance are expensive.

SUMMARY OF THE INVENTION

The above problems of prior art have been substantially eliminated with a spunbond nonwoven web for an acquisition/distribution layer, the web comprising at least

- a first layer of filaments, wherein the first layer consists of continuous crimped bi-component filaments of an eccentric core /sheath structure, the filaments having a diameter in the range of 15 to 35 microns and exhibiting at least 3 crimps/cm, wherein the core of the filaments consists of a material having a higher melting point than the sheath,
- a second filament layer arranged in direct contact on the first layer, wherein the second layer of filaments comprises continuous crimped bi-component filaments of an eccentric core /sheath structure, the filaments having a diameter, which is smaller than the diameter of the filaments in the first layer and which is in the range of 10 to 20 microns and exhibiting at least 3 crimps/cm, wherein the core consists of a material having a higher melting point than the sheath.

It has been found that the spunbond nonwoven web according to the invention has a very satisfactory acquisition/distribution properties as well as rewet characteristics.

Preferably, at least some of the filaments of the second layer are mutually thermally bonded via their sheath components and at least some of the filaments of the first and the second layer are mutually thermally bonded together via their sheath components.

Preferably, the core of the filaments of the first layer and/or of the second filament layer consists of PET or PLA or PET copolymer and extends over at least 50 % of the filament cross section.

The sheath of the filaments of the first layer and/or of the second layer may comprise polyethylene homopolymer or polyethylene copolymer or polypropylene copolymer.

Also preferably, the first layer has a lower bulk density than the second layer, wherein preferably the difference of bulk density is 5 to 80 kg/m$^3$, preferably 40 to

60 kg/m$^3$.

The degree of bonding at the outer surface of the second layer preferably decreases continually through the nonwoven web in the direction towards the outer surface of the first layer.

According to a specific preferred embodiment, the first layer consists of continuous crimped bi-component filaments having a diameter of 20 to 30 microns, and showing 5-15 crimps/cm, and/or the second layer consists of continuous crimped bi-component filaments having a diameter of 15 to 20 microns and showing 5-15 crimps/cm.

The second filament layer may be shrunken and/or the first filament layer may comprise wrinkles at its outer surface, at least 30% of which form with the machine direction an angle which is in the range of 5 and 20°.

The problems of prior art are eliminated to an extensive degree by an absorbent article comprising an acquisition/distribution layer consisting of the above spunbond nonwoven web, a liquid pervious topsheet, a liquid impervious backsheet joined to said topsheet; an absorbent core positioned between said topsheet and said backsheet, wherein the acquisition/distribution layer is positioned between said topsheet and said absorbent core.

Preferably, the acquisition/distribution layer has a length and a width, the filaments extending substantially along the length direction and having a length which is at least 120%, preferably at least 150% most preferably at least 180% the length of the length of the acquisition/distribution layer.

For ADL structures hydrophilicity is considered important. Therefore, a hydrophilicity enhancing additive may be preferably added to the sheath component of the filaments. Alternatively or additionally, it is possible to impregnate the nonwoven web subsequently with a hydrophilic surfactant (e.g. Silastol PHP 90 from Schill and Seilacher) using a dip roller (kiss-roll), foulard, etc.

Preferably the difference between the average diameters of the filaments of the first layer and the filaments of the second layer is equal to or more than 2 microns, preferably in the range of about 2 to 7 microns.

Preferably the second layer has a higher value of bulk density than the first layer and the difference between bulk density of the second layer and the bulk density of the first layer is equal to or more than 5 kg/m$^3$, preferably in the range of about 5 to 80 kg/m$^3$, more preferably between 40 to 60 kg/m$^3$.

Preferably, an acquisition layer formed by said 2 layer laminate according to the invention has a caliper within the range of 0.5 - 3 mm, preferably 0.7 mm to 1.5 mm.

Preferably, the method of producing the nonwoven web according to the invention comprises the step of dimensional consolidation, i.e. shrinking the second layer such that the second layer assumes an area forming about 95 % to 70 % of its initial area.

A specific advantageous embodiment of the invention is an absorbent article comprising a liquid pervious topsheet, a liquid impervious backsheet joined to said topsheet; an absorbent core positioned between said topsheet and said backsheet, and the acquisition/distribution layer according to the invention positioned between said topsheet and said absorbent core. The ADL is positioned such that the first layer faces the topsheet and the second layer faces the absorbent core. In such embodiment the ADL will have a defined length typically, but not necessarily, being shorter than the absorbent core of the absorbent hygienic product. The even more advantageous embodiment of this invention will be an ADL layer consisting of filaments which are at least 20% longer (preferably at least 30 % longer) than the length of the ADL.

## Definitions

The term **"acquisition/distribution layer"** or **"ADL"** refers to a material layer typically in an absorbent hygiene product, typically a nonwoven, between the topsheet and the absorbent core. These layers are designed to quickly acquire and/or distribute the fluid away from the topsheet and into the core. These layers are sometimes called "wicking layer", "surge layer", "acquisition layer" or "distribution layer". Articles having an ADL layer consisting of only one sub-layer (a bonded batt) are known. Articles having two sub-layers or more are also known. Ideally one sub-layer shall mainly pull the fluid quickly away from the topsheet and distribute the fluid in the direction towards the core and also in other the directions throughout the layer and the other sub-layer should lower the tendency of fluid travel from the core towards the first sub-layer and towards the topsheet, i.e. to lower or prevent rewetting of topsheet. These sub-layers typically do not comprise superabsorbent material. In the following, the term "acquisition-distribution layer" ("ADL") will be used to designate the layer present between the topsheet and the absorbent core providing these acquisition and distribution functions, irrespective of the number of fiber sub-layers (batts) forming the layer.

The term **"batt"** refers to materials in the form of filaments that are found in the state prior to bonding that is performed during the calendering process described for example in patent application WO2012130414. The "batt" consists of individual filaments between which a fixed mutual bond is usually not yet formed even though they may be pre-bonded in certain ways, where this pre-bonding may occur during or shortly after the laying of filaments in the spunlaying process. This pre-bonding, however, still permits a substantial number of the filaments to be freely moveable such that they can be repositioned. The above mentioned "batt" may consist of several strata created by the deposition of filaments from several spinning beams in the spunlaying process.

The term **"filament"** refers to a principally endless fiber while the term **"staple fiber"** refers to a fiber which has been cut to a defined length.

The term **"monocomponent filament"** refers to a filament formed of a single polymer or polymer blend, as distinguished from bicomponent or multicomponent filament.

**"Bicomponent filament"** refers to a filament having a cross-section comprising two discrete polymer sections, two discrete polymer blend sections, or one discrete polymer section and one discrete polymer blend section. The term "bicomponent filament" is encompassed within the term "multicomponent filament". A bicomponent filament may have an overall cross-section divided into two or more sections consisting of differing sections of any shape or arrangement, including for example, a coaxial arrangement, core-and-sheath arrangement, side-by-side arrangement, radial arrangement, etc.

A bicomponent filament having a **"core/sheath structure"** has a cross-section comprising two discrete polymer or polymer blend sections, wherein the sheath polymer or polymer blend component is disposed around the core polymer or polymer blend component.

The term **"eccentric core/sheath"** structure refers to a filament having a cross-section, in which the center of gravity of the core component is offset from the center of gravity of the filament. When the sheath component has different solidification characteristics than the core component, especially when the sheath component has a melting point at least 20°C higher than the melting point of the core component, such a structure promotes crimping of the filament.

**"Spunbond"** nonwovens are made in one continuous process. Filaments are spun and then directly dispersed onto a continuously moving belt.

As used herein, the term **"nonwoven textile"** means a structure in the form of a fleece or webbing formed from directed or randomly oriented filaments, from which initially a batt is formed and which is subsequently consolidated and filaments are mutually bonded by friction, effects of cohesive forces, gluing or by similar methods creating a single or multiple bonding patterns consisting of bonding imprints formed by a bounded compression and/or the effect of pressure, heat, ultrasound or heat energy, or a combination of these effects if necessary. The term does not refer to fabrics formed by weaving or knitting or fabrics using yarn or filaments to form bonding stitches.

Filaments forming the first layer and the second layer according to the invention are oriented substantially in the machine direction. As the filaments are crimped and continuous (i.e. endless), it means that most filaments as a whole extend substantially in the machine direction, although- as a result of their crimps - they comprise segments which extend along directions differing from the machine direction more or less significantly.

FIGURES

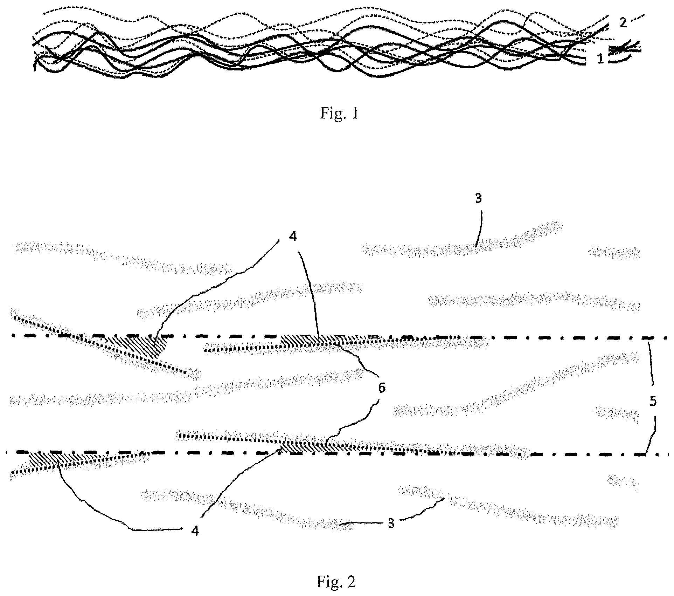

Fig. 1: Material according to the invention: the web comprising at least a first layer of thicker crimped filaments and a second layer of thinner crimped filaments

Fig. 2: Material according to the invention: the wrinkles forming angles with machine direction

Fig 3-1 to 3-3: Examples of crimp counting on fibres in bonded fabric.

DETAILED DESCRIPTION OF PREFERRED EMBODIMENTS

Example 1 - eC/S PET /PE homopolymer 70/30

A spunbond type nonwoven web is produced via a continuous process using two beams. Each of the beams is fed a composition that consists essentially of about 70% by weight of a polyethylene terephthalate homopolymer (5520 from Invista), 30% by weight of polyethylenelene homopolymer (Aspun 6834from DOW). For both beams the temperature of polymeric composition measured after the extruder zone is between between 270-300 °C for PET and 230-235 °C for PE. Melt spun bicomponent filaments with diameter of 16 $\mu$m and with filament composition core/sheet are produced with filament speed 3000 m/min and subsequently collected on a conveyor belt continuously moving by speed 190 m/min. The cross section of the filament comprises a core, which consists of a polyethylene terephthalate homopolymer, and a sheath which surrounds the core and consists of a polyethylene homopolymer, the core being arranged eccentrically with respect to the outer surface of the sheath, i.e. the center of gravity of the core is offset from the center of gravity of the filament cross section. Filaments are conveyed through continuous narrow flow of air with temperature 130°C to enhance and/or set their crimp. The basis weight of the nonwoven layer thus formed is 35 gsm and the bulk density about 105 kg/m$^3$.

Example 2 - eC/S PET/PE homopolymer 70/30

A spunbond type nonwoven web is produced via a continuous process using two beams. Each of the beams is fed a composition that consists essentially of about 70% by weight of a polyethylene terephthalate homopolymer (5520 from Invista), 30% by weight of polyethylenelene homopolymer (Aspun 6834from DOW). For both beams the temperature of polymeric composition measured after the extruder zone is between between 270-300 °C for PET and 230-235 °C for PE. Melt spun bicomponent filaments with diameter of 20 $\mu$m and with filament composition core/sheet are produced with filament speed 2000 m/min and subsequently collected on a conveyor belt continuously moving by speed 190 m/min.The cross section of the filament comprises a core, which consists of a polyethylene terephthalate homopolymer, and a sheath which surrounds the core and consists of a polyethylene homopolymer, the core being arranged eccentrically with respect to the outer surface of the sheath, i.e. the center of gravity of the core is offset from the center of gravity of the filament cross section. Filaments are conveyed through continuous narrow flow of air with temperature 140 °C to enhance and/or set their crimp. The basis weight of the nonwoven layer thus formed is 35 gsm and the bulk density about 56 kg/m$^3$.

Example 3 - eC/S PET/PE homopolymer 70/30

A spunbond type nonwoven web is produced via a continuous process using two beams. Each of the beams is fed a composition that consists essentially of about 70% by weight of a polyethylene terephthalate homopolymer (5520 from Invista), 30% by weight of polyethylene homopolymer (Aspun 6834 from DOW). For both beams the temperature of polymeric composition measured after the extruder zone is between between 270-300 °C for PET and 230-235 °C for PE. Melt spun bicomponent filaments with diameter of 15 μm and with filament composition core/sheet are produced with filament speed 3000 m/min and subsequently collected on a conveyor belt continuously moving by speed 267 m/min .The cross section of the filament comprises a core, which consists of a polyethylene terephthalate homopolymer, and a sheath which surrounds the core and consists of a polyethylene homopolymer, the core being arranged eccentrically with respect to the outer surface of the sheath, i.e. the center of gravity of the core is offset from the center of gravity of the filament cross section. Filaments are conveyed through continuous narrow flow of air with temperature 140 °C to enhance and/or set their crimp. The basis weight of the nonwoven layer thus formed is 25 gsm, the bulk density is about 107 kg/m$^3$.

Example 4 - eC/S PET/PE homopolymer 70/30

A spunbond type nonwoven web is produced via a continuous process using two beams. Each of the beams is fed a composition that consists essentially of about 70% by weight of a polyethylene terephthalate homopolymer (5520 from Invista), 30% by weight of polyethylene homopolymer (Aspun 6834 from DOW). For both beams the temperature of polymeric composition measured after the extruder zone is between between 270-300 °C for PET and 230-235 °C for PE. Melt spun bicomponent filaments with diameter of 21 μm and with filament composition core/sheet are produced with filament speed 2000 m/min and subsequently collected on a conveyor belt continuously moving by speed 267 m/min. The cross section of the filament comprises a core, which consists of a polyethylene terephthalate homopolymer, and a sheath which surrounds the core and consists of a polyethylene homopolymer, the core being arranged eccentrically with respect to the outer surface of the sheath, i.e. the center of gravity of the core is offset from the center of gravity of the filament cross section. Filaments are conveyed through continuous narrow flow of air with temperature 140 °C to enhance and/or set their crimp. The basis weight of the nonwoven layer thus formed is 25 gsm, the bulk density is about 60 kg/m$^3$.

Example 5 - eC/S PET/PE homopolymer 70/30

A spunbond type nonwoven web is produced via a continuous process using two beams. Each of the beams is fed a composition that consists essentially of about 70% by weight of a polyethylene terephthalate homopolymer (5520 from Invista), 30% by weight of polyethylene homopolymer (Aspun 6834 from DOW). For both beams the temperature of polymeric composition measured after the extruder zone is between between 270-300 °C for PET and 230-235 °C for PE. Melt spun bicomponent filaments with diameter of 12 μm and with filament composition core/sheet are produced with filament speed 5000 m/min and subsequently collected on a conveyor belt continuously moving by speed 267 m/min. The cross section of the filament comprises a core, which consists of a polyethylene terephthalate homopolymer, and a sheath which surrounds the core and consists of a polyethylene homopolymer, the core being arranged eccentrically with respect to the outer surface of the sheath, i.e. the center of gravity of the core is offset from the center of gravity of the filament cross section. Filaments are conveyed through continuous narrow flow of air with temperature 140 °C to enhance and/or set their crimp. The basis weight of the nonwoven layer thus formed is 25 gsm, the bulk density is about 110 kg/m$^3$.

Example 6 - eC/S PET/PE homopolymer 70/30

A spunbond type nonwoven web is produced via a continuous process using two beams. Each of the beams is fed a composition that consists essentially of about 70% by weight of a polyethylene terephthalate homopolymer (5520 from Invista), 30% by weight of polyethylene homopolymer (Aspun 6834 from DOW). For both beams the temperature of polymeric composition measured after the extruder zone is between between 270-300 °C for PET and 230-235 °C for PE. Melt spun bicomponent filaments with diameter of 16 μm and with filament composition core/sheet are produced with filament speed 3000 m/min and subsequently collected on a conveyor belt continuously moving by speed 267 m/min .The cross section of the filament comprises a core, which consists of a polyethylene terephthalate homopolymer, and a sheath which surrounds the core and consists of a polyethylene homopolymer, the core being arranged eccentrically with respect to the outer surface of the sheath, i.e. the center of gravity of the core is offset from the center of gravity of the filament cross section. Filaments are conveyed through continuous narrow flow of air with temperature 140 °C to enhance and/or set their crimp. The basis weight of the nonwoven layer thus formed is 25 gsm, the bulk density is about 115 kg/m$^3$.

Example 7 - eC/S PLA/PE homopolymer 50/50

A spunbond type nonwoven web is produced via a continuous process using two beams. Each of the beams is fed a composition that consists essentially of about 50% by weight of a polylactic acid homopolymer (6302D from Nature Works), 50% by weight of polyethylene homopolymer (Aspun 6834from DOW). For both beams the temperature of polymeric composition measured after the extruder zone is about 230 °C for PLA and about 230 °C for PE. Melt spun bicomponent filaments with diameter of 15 $\mu$m and with filament composition core/sheet are produced with filament speed 3000 m/min and subsequently collected on a conveyor belt continuously moving by speed 267 m/min. The cross section of the filament comprises a core, which consists of a polylactic acid homopolymer, and a sheath which surrounds the core and consists of a polyethylene homopolymer, the core being arranged eccentrically with respect to the outer surface of the sheath, i.e. the center of gravity of the core is offset from the center of gravity of the filament cross section.
Filaments are conveyed through continuous narrow flow of air with temperature 107°C to enhance and/or set their crimp. The basis weight of the nonwoven layer thus formed is 25 gsm and bulk density about 102 kg/m$^3$.

Example 8 - eC/S PLA/PE homopolymer 50/50

A spunbond type nonwoven web is produced via a continuous process using two beams. Each of the beams is fed a composition that consists essentially of about 50% by weight of a polylactic acid homopolymer (6302D from Nature Works), 50% by weight of polyethylene homopolymer (Aspun 6834from DOW). For both beams the temperature of polymeric composition measured after the extruder zone is about 230 °C for PLA and about 230 °C for PE. Melt spun bicomponent filaments with diameter of 20 $\mu$m and with filament composition core/sheet are produced with filament speed 2000 m/min and subsequently collected on a conveyor belt continuously moving by speed 267 m/min. The cross section of the filament comprises a core, which consists of a polylactic acid homopolymer, and a sheath which surrounds the core and consists of a polyethylene homopolymer, the core being arranged eccentrically with respect to the outer surface of the sheath, i.e. the center of gravity of the core is offset from the center of gravity of the filament cross section.
Filaments are conveyed through continuous narrow flow of air with temperature 107°C to enhance and/or set their crimp. The basis weight of the nonwoven layer thus formed is 25 gsm and bulk density about 55 kg/m$^3$.

Example 9 - eC/S PET/PE copolymer 70/30

Spunbond nonwoven textile has been produced in the same way as Example 3, except that polyethylene copolymer has been used in the sheath instead of polyethylene homopolymer.

Example 10 - eC/S PET/PE copolymer 70/30

Spunbond nonwoven textile has been produced in the same way as Example 4, except that polyethylene copolymer has been used in the sheath instead of polyethylene homopolymer.

Example 11 - eC/S PET/PP copolymer 70/30

Spunbond nonwoven textile has been produced in the same way as Example 3, except that polypropylene copolymer has been used in the sheath instead of polyethylene homopolymer.

Example 12 - eC/S PET/PP copolymer 70/30

Spunbond nonwoven textile has been produced in the same way as Example 4, except that polypropylene copolymer has been used in the sheath instead of polyethylene homopolymer.
According to the invention the spubond nonwoven comprises two layers 1, 2 one arranged in direct contact on the other. Superimposing one layer on the other may be done in various ways. According to one embodiment a layer 2 (e.g. Example 1) is directly deposited onto another layer 1 (e.g. Example 2) within an in-line production process and subsequently the composite is bonded or at least consolidated by leading the composite through a hot air bonding unit (hot-air knife or hot air diffuser), which blows hot-air at the upper surface of the composite and - if required - concurrently or subsequently at the lower surface of the composite. The sheath components of the filaments partially melt during the process, so that they bond the filaments together at the points where they contact each other.
According to another embodiment, at least one of the layers 1, 2, preferably both layers 1, 2 are at least partially pre-bonded to facilitate handling. Then, the pre-bonded layers 1, 2 are superimposed and consolidated by means of the above mentioned hot air bonding unit.
According to a preferred embodiment, a first layer 1 of crimped bi-component filaments having an eccentric core/sheath structure and a diameter of about 15 to 35 microns is provided and a second layer 2 of crimped bi-component filaments is deposited onto the first layer 1, the second layer filaments having an eccentric core/sheath structure, and a diameter lower than the filaments of the first layer 1 and in the range of about 10 to 20 microns. The second layer 2 has a density higher than the first layer 1. The composite is exposed to a hot air knife or hot air diffuser such that the hot air impacts the second filament layer 2, the temperature of the hot air impacting the second filament layer 2 being slightly above the melting point of the sheath component of the filaments of the second layer 2. As the hot air impacts the outer surface of the second filament layer 2 and then passes through the composite, the sheath component of the filaments get tacky or melt and the filaments are fused at the points of their cross-section. The whole composite is thermally bonded in such a manner that the farther from the outer surface of the second layer 2, the lower degree of bonding. Without intending to be bound by theory, it is believed that this is due to i) the decrease of temperature of the hot air as the air travels from the point of impact through the composite towards the outer surface of the first layer 1, and ii) higher surface to mass ratio of the filaments of the second filament layer 2, because of the filaments having lower diameter than the filaments of the first filament layer 1.

Thus the resulting composite or laminate comprises the first layer 2 of coarser filaments having a lower density and the second layer 2 of finer filaments having a higher density, while the degree of bonding (and the number of bonding points) decreases from the outer surface of the second layer 2 towards the contact surface between the first layer 1 and the second layer 2 and therefrom towards the outer surface of the first layer 1. The total volume of voids within the nonwoven decreases significantly in the opposite direction.

According to the invention, the first layer 1 of crimped bi-component filaments has an eccentric core/sheath structure and a diameter of more than about 15 microns.

According to the invention, the first layer 1 of crimped bi-component filaments has an eccentric core/sheath structure and a diameter of less than about 35 microns, preferably less than about 30 microns, preferably less than about 28 microns, preferably less than about 26 microns, most preferably less than about 24 microns.

According to the invention, the second layer 2 of crimped bi-component filaments has an eccentric core/sheath structure and a diameter of more than about 10 microns, preferably more than about 11 microns, most preferably more than about 12 microns.

According to the invention, the second layer 2 of crimped bi-component filaments has an eccentric core/sheath structure and a diameter of less than about 20 microns, preferably less than about 18 microns, most preferably less than about 16 microns.

Preferably, the difference in fiber diameter between first layer 1 and the second layer 2 is more than 2 microns, preferably more than 3 microns, preferably more than 4 microns.

Preferably, the difference in fiber diameter between the first layer 1 and the second layer 2 is less than 20 microns, preferably less than 16 microns, preferably less than 13 microns, preferably less than 10 microns.

According to the invention, the first layer 1 consists of fibers showing at least 3 crimps/cm, preferably at least 4 crimps/cm, more preferably at least 5 crimps/cm, most preferably at least 6 crimps/cm.

Preferably, the first layer 1 consists of fibers showing max. 30 crimps/cm, preferably max. 25 crimps/cm, more preferably max 20 crimps/cm, most preferably max 15 crimps/cm. According to the invention, the second layer 2 consist of fibers showing at least 3 crimps/cm, preferably at least 4 crimps/cm, more preferably at least 5 crimps/cm, more preferably at least 6 crimps/cm, more preferably at least 8 crimps/cm, most preferably at least 10 crimps/cm.

Preferably, the second layer 2 consists of fibers showing max. 30 crimps/cm, preferably max. 25 crimps/cm, more preferably max. 20 crimps/cm, most preferably max. 15 crimps/cm. In another advantageous embodiment, the second layer 2 consists of fibers showing 5 - 15 crimps/cm and the first layer 1 consists of fibers showing 5-15 crimps/cm. In another embodiment it can be preferred, when the level of crimping in the first layer 1 is lower than the level of crimping in the second layer 2.

In another embodiment it can be preferred, when the level of crimping in the first and second layer 1, 2 is more or less the same. A person skilled in the art will know that due to various technological issues it can occur during the production process that some individual fibers are not crimped in the desired way. Not to be bound by theory, we expect to achieve an inventive product that behaves in the described way with at least 90% of the fibers crimped according to the description. Additionally, the second layer 2 of filaments may shrink due to the application of heat from the hot air knife or diffuser. The first layer 1, which has not shrunk or which has shrunken less than the second layer 2, becomes more lofty then. This is because the second layer 2 has been bonded to the first layer 1 by the heat, however, as the heat was not enough to initiate shrinking of the first layer 1, the first layer 1 wrinkled as the second layer 2 shrunk. The wrinkles 3 and their directions are neither strictly regularly distributed nor oriented, they form channels extending mostly about in the machine direction 5, but at least 30%, preferably at least 40 % of the wrinkles 3 forms an angle 4 with the machine direction 5, the angle 4 being in the range of 5° to 20°. The resulting nonwoven textile has a wrinkled first surface provided by the first layer 1 and a substantially even second surface provided by the second layer 2. This effect may also appear when both layers 1, 2 shrink, but with a different level of shrinkage for each of the layer.

When such a composite is used as an acquisition / distribution layer within an absorbent article, the wrinkles 3 and their varying direction at the surface of the ADL enhance the ability of the ADL to distribute liquid in various parts of the core of the absorbent article. According to the invention, the spunbond nonwoven layers 1, 2 comprise crimped fibers. The formation of such crimped fibers resulting from a significant difference in the properties of the individual subsections, commonly expressed using the so-called contractibility of individual subsections is well known in the industry. Fibers produced in this way are known under the name of chemically formed fibers. A person skilled in the art will appreciate that the term subsection contractibility describes primarily the volume during the transition from the liquid to the solid state, which is affected by the various properties of the polymers. A person skilled in the art will also appreciate that the difference in crystallinity can also be a crimping engine. The growth rate of crystals and the level of crystalinity can cause so-called "secondary crimping" when the fiber is in the solid state. Crimping can be also activated or supported by added energy (for. example heating, mechanical force, etc.). A person skilled in the art will appreciate that crimping theories are described in literature and the above mentioned cases are examples of possible principles.

According to the invention, the nonwoven can comprise bicomponent eccentric C/S fibers, wherein the core comprises polyesters including both artificial ones (as for example Polyethylene Terephthalate "PET") and bio-based ones (such as for example Polylactic Acid - "PLA") or their copolymers. In some embodiments, it may be preferred to have the same core material in both the first and the second layer 1, 2. In other embodiments it may be preferred to combine the core materials in the final product - e.g. to have PLA as a core material in the first layer 1 with thicker fibers and PET as a core material in the second layer 2 with thinner fibers. A person skilled in the art can appreciate that different core polymers can lead to different final properties of the layer surface (for example stiffness). The final product can be designed according to specific needs. According to the invention, the nonwoven can comprise bicomponent eccentric C/S fibers, where the sheath comprises polymers with a lower melt temperature than the core polymer. With advantage the polymers can be chosen from a group of polyolefins (e.g. polypropylene - "PP" or polyethylene - "PE") or their copolymers (e.g. polypropylene copolymer - "coPP", polyethylene copolymer - "coPE"). With advantage, polymers can also be chosen from a group of polyester copolymers (e.g. polyethylene teraphtalate copolymer - "coPET").

A person skilled in the art can also appreciate the advantage of the use of polymer blends from above mentioned groups or of adding suitable additives.

In some embodiments, it can be advantageous to have the same sheath material in both the first and the second layer 1, 2. In other embodiments it can be preferred to combine the sheath material in the final material - e.g. to have the PP sheath in the first layer and the coPP sheath in the second layer. The sheath material in the first and the second layer 1,2 shall show very good mutual miscibility. A person skilled in the art will appreciate, for example, the different level of adhesion on the outer surface of the first and the second layer 1, 2 that can be used with advantage, for example, in its positioning in a hygiene absorbent product. According to the invention, the nonwoven can be with advantage used as a part of a hygiene disposable product, as a diaper, feminine care product, adult incontinence product, absorbent pad or other types of absorbent products, where an acquisition distribution function is desired. Modern absorbent products are constructed as multilayer structures, where the neighboring functional layers may with advantage be so perfectly adjacent to one another that they seem to associate. The nonwoven according to the invention shall be generally placed between the topsheet and the absorbent core. The topsheet can be designed, for example, from a single layer to create a multilayer soft bulky structure with pleasant touch characteristics. The absorbent core can be constructed in different ways, for example, from a fluffy core with a low amount of superabsorbent or no superabsorbent to a so called fluffless core with a high amount of superabsorbent. The absorbent core can be also covered in so-called corewrap. A person skilled in the art will appreciate that the nonwoven according to the invention can be used as an ADL functional layer or as a part of an ADL functional layer, being combined, for example, with some other layer according to the absorbent product designer's intention.

**Specific examples of spunbond nonwoven web according to the invention**

Example 13

Examples 1 and 2 were combined laminating Example 2 onto Example 1 to form a double layer nonwoven structure, wherein the layer consisting of Example 1 formed the second layer 2 and consisted of filaments having a diameter of about 16 microns and crimping degree about 13 crimps/cm and bulk density about 105 kg/m$^3$ and the layer consisting of Example 2 formed the first layer 1 and consisted of filaments having a diameter of about 20 microns and crimping degree about 7 crimps/cm and bulk density about 56 kg/m$^3$. Thus a composite is provided, having a total basis weight of 70 gsm and thickness of about 1 mm. The layers 1, 2 have been bonded together by leading the composite through a hot air bonding unit (hot-air knife or hot air diffuser), which blew hot-air at the outer surface of the second layer 2, the hot-air having a temperature of about 125 - 130 °C at impacting the second layer 2. The duration of impact and the velocity/pressure of the hot air were such that the sheaths of the filaments at the outer surface of the second layer 2 have been melted enough to provide strong bonding, while the filaments at the outer surface of the first layer 1 remained substantially unbonded. In this specific embodiment, the hot-air impact at a specific point lasted 2,5 seconds in hot air dryer - Size. 1 / Ø2600 - AB 4800 (FLEISSNER GmbH) with air circulation set on 90 % and air exhaust set on 100 %. During such bonding of the layers 1, 2, the hot air (e.g. from omega Fleissner dryer) impacts the outer surface of the second layer 2 and penetrates through the layers 1, 2 and the temperature of the air drops down gradually from the point of impact, such that the temperature of the air acting upon the first layer 1 is lower than the temperature of the air acting upon the second layer 2. Thus, an effect of different shrinkage of the two individual layers 1, 2 may be generated. The layer, which has shrunken less, can generate a channels-wrinkles like 3 D structure and this positively improves bulkiness of the nonwoven web and also its liquid storage capacity and liquid distribution ability.

Example 14

Examples 3 and 4 were combined laminating Example 4 onto Example 3 to form a double layer nonwoven structure, wherein the layer consisting of Example 3 formed the second layer 2 and consisted of filaments having a diameter of about 15 microns and crimping degree about 14 crimps/cm and bulk density about 107 $kg/m^3$ and the layer consisting of Example 4 formed the first layer 1 and consisted of filaments having a diameter of about 21 microns and crimping degree about 8 crimps/cm and bulk density about 60 $kg/m^3$. Thus a composite is provided, having a total basis weight of 50 gsm and thickness of about 1 mm. The layers 1, 2 have been bonded together by leading the composite through a hot air bonding unit (hot-air knife or hot air diffuser), which blew hot-air at the outer surface of the second layer 2, the hot-air having a temperature of about 125 - 130 °C at impacting the second layer 2. The duration of impact and the velocity/pressure of the hot air were such that the sheaths of the filaments at the outer surface of the second layer 2 have been melted enough to provide strong bonding, while the filaments at the outer surface of the first layer 1 remained substantially unbonded. In this specific embodiment, the hot-air impact at a specific point lasted about 2 seconds in hot air dryer - Size. 1 / Ø2600 - AB 4800 (FLEISSNER GmbH) with air circulation set on 90 % and air exhaust set on 100 %. Again, during such bonding of the layers 1, 2, the hot air (e.g. from omega Fleissner dryer) impacts the outer surface of the second layer 2 and penetrates through the layers 1, 2 and the temperature of the air drops down gradually from the point of impact, such that the temperature of the air acting upon the first layer 1 is lower than the temperature of the air acting upon the second layer 2. Thus, an effect of different shrinkage of the two individual layers 1, 2 may be generated. The layer, which has shrunken less, can generate a channels-wrinkles like 3 D structure and this positively improves bulkiness of the nonwoven web and also its liquid storage capacity and liquid distribution ability.

Example 15

Examples 5 and 6 were combined laminating Example 6 onto Example 5 to form a double layer nonwoven structure, wherein the layer consisting of Example 5 formed the second layer 2 and consisted of filaments having a diameter of about 12 microns and crimping degree about 4 crimps/cm and bulk density about 110 $kg/m^3$ and the layer consisting of Example 6 formed the first layer 1 and consisted of filaments having a diameter of about 16 microns and crimping degree about 6 crimps/cm and bulk density about 115 $kg/m^3$. Thus a composite is provided, having a total basis weight of 50 gsm and thickness of about 1 mm. The layers 1, 2 have been bonded together by leading the composite through a hot air bonding unit (hot-air knife or hot air diffuser), which blew hot-air at the outer surface of the second layer 2, the hot-air having a temperature of about 125 - 130 °C at impacting the second layer 2. The duration of impact and the velocity/pressure of the hot air were such that the sheaths of the filaments at the outer surface of the second layer 2 have been melted enough to provide strong bonding, while the filaments at the outer surface of the first layer 1 remained substantially unbonded. In this specific embodiment, the hot-air impact at a specific point lasted 1,5 seconds.

Example 16

Examples 7 and 8 were combined laminating Example 8 onto Example 7 to form a double layer nonwoven structure, wherein the layer consisting of Example 7 formed the second layer 2 and consisted of filaments having a diameter of about 15 microns and crimping degree about 13 crimps/cm and bulk density about 102 $kg/m^3$ and the layer consisting of Example 8 formed the first layer 1 and consisted of filaments having a diameter of about 20 microns and crimping degree about 7 crimps/cm and bulk density about 55 $kg/m^3$. Thus a composite is provided, having a total basis weight of 50 gsm and thickness of about 1 mm. The layers 1, 2 have been bonded together by leading the composite through a hot air bonding unit (hot-air knife or hot air diffuser), which blew hot-air at the outer surface of the second layer, the hot-air having a temperature of about 125 - 130 °C at impacting the second layer 2. The duration of impact and the velocity/pressure of the hot air were such that the sheaths of the filaments at the outer surface of the second layer 2 have been melted enough to provide strong bonding, while the filaments at the outer surface of the first layer 1 remained substantially unbonded. In this specific embodiment, the hot-air impact at a specific point lasted 2,5 seconds in hot air dryer - Size. 1 / Ø2600 - AB 4800 (FLEISSNER GmbH) with air circulation set on 90 % and air exhaust set on 100 %. Again, during such bonding of the layers, different shrinkage of the individual layers may be achieved, as described in respect of Example 13 and 14.

Example 17

Examples 9 and 10 were combined laminating said Example 10 onto said Example 9 to form a double layer nonwoven structure, wherein the layer consisting of said Example 9 formed the second layer 2 and consisted of filaments having a diameter of about 15 microns and crimping degree about 12 crimps/cm and bulk density about 108 kg/m$^3$ and the layer consisting of said Example 10 formed the first layer 1 and consisted of filaments having a diameter of about 21 microns and crimping degree about 6 crimps/cm and bulk density about 59 kg/m$^3$. Thus a composite is provided, having a total basis weight of 50 gsm and thickness of about 1 mm. The layers 1, 2 have been bonded together by leading the composite through a hot air bonding unit (hot-air knife or hot air diffuser), which blew hot-air at the outer surface of the second layer 2, the hot-air having a temperature of about 125 - 130 °C at impacting the second layer 2. The duration of impact and the velocity/pressure of the hot air were such that the sheaths of the filaments at the outer surface of the second layer 2 have been melted enough to provide strong bonding, while the filaments at the outer surface of the first layer 1 remained substantially unbonded. In this specific embodiment, the hot-air impact at a specific point lasted 2 seconds in hot air dryer - Size. 1 / Ø2600 - AB 4800 (FLEISSNER GmbH) with air circulation set on 90 % and air exhaust set on 100 %. Again, during such bonding of the layers, different shrinkage of the individual layers may be achieved, as described in respect of Example 13 and 14.

Example 18

Examples 11 and 12 were combined laminating laminating said Example 12 onto said Example 11 to form a double layer nonwoven structure, wherein the layer consisting of said Example 11 formed the second layer 2 and consisted of filaments having a diameter of about 16 microns and crimping degree about 13 crimps/cm and bulk density about 106 kg/m$^3$ and the layer consisting of said Example 12 formed the first layer 1 and consisted of filaments having a diameter of about 20 microns and crimping degree about 6 crimps/cm and bulk density about 58 kg/m$^3$. Thus a composite is provided, having a total basis weight of 50 gsm and thickness of about 1 mm. The layers 1, 2 have been bonded together by leading the composite through a hot air bonding unit (hot-air knife or hot air diffuser), which blew hot-air at the outer surface of the second layer 2, the hot-air having a temperature of about 125 - 130 °C at impacting the second layer 2. The duration of impact and the velocity/pressure of the hot air were such that the sheaths of the filaments at the outer surface of the second layer 2 have been melted enough to provide strong bonding, while the filaments at the outer surface of the first layer 1 remained substantially unbonded. In this specific embodiment, the hot-air impact at a specific point lasted 2 seconds in hot air dryer - Size. 1 / Ø2600 - AB 4800 (FLEISSNER GmbH) with air circulation set on 90 % and air exhaust set on 100 %. Again, during such bonding of the layers, different shrinkage of the individual layers may be achieved, as described in respect of Example 13 and 14.

**Test methods:**

The **"Basis weight"** of a nonwoven web is measured according to the European standard test EN ISO 9073-1:1989 (conforms to WSP 130.1). There are 10 nonwoven web layers used for measurement, Example size 10x10 cm2.

The **"Caliper"** or **"Thickness"** of the nonwoven material is measured according to the European standard test EN ISO 9073-2:1995 (conforms to WSP 120.6) with following modification:

1. the material shall be measured on an Example taken from production without being exposed to higher strength forces or spending more than a day under pressure (for. example on a product roll), otherwise before measurement the material has to lie freely on a surface for at least 24 hours.

2. the overall weight of upper arm of the machine including added weight is 130 g.

The **"degree of crimping"** is measured according to ASTM D-3937-82 with following modification:

1.as unit of measurement is used "crimps/cm"
Setting the degree of crimping in a bonded layer is an issue since single fibres are bonded to each other and it is not possible to remove one of them from the composition (without the danger of affecting the original crimp level) and to measure the crimp value and the fiber length. For the purpose of this invention, the following estimation may be used:

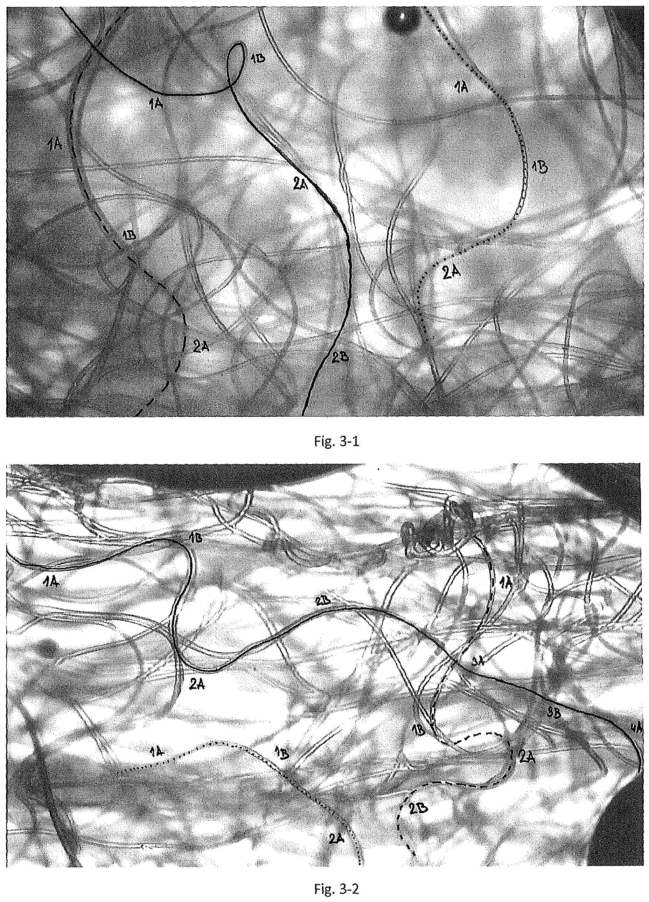

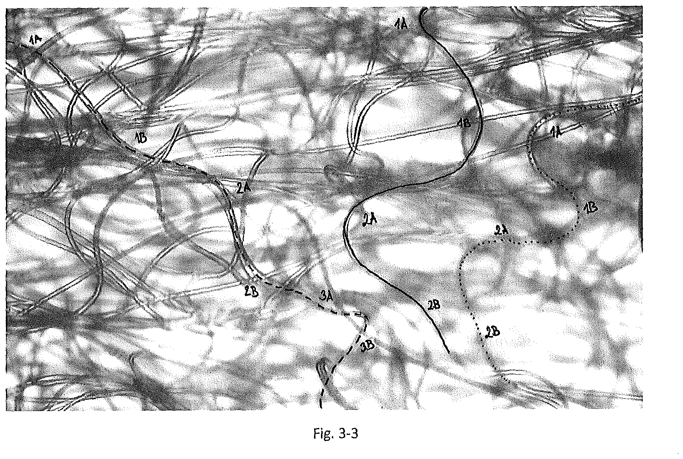

1) A picture of the assessed layer is provided in such a magnification that the fibers can be well seen
2) One single fiber is chosen and its path through the picture or at least part of the picture is marked
3) The length of the marked fiber in the picture is measured
4) The number of crimps in the measured length is counted
In contrast to the measurement of individual fibers, it is not possible to place the fiber in such a way that all the crimps can be seen clearly and then counted in a repeating sequence. In a bonded structure, some parts can be masked in the z direction, some parts may be masked by other fibers; some parts may be masked by bonding. Each fiber turn shall be counted as half a crimp. Also, a change from sharp to blurry on one fiber shall be counted as half a crimp - see the examples at Fig. 3-1 to 3-3, where the half-crimps 1A + IB form one crimp (the same applies to 2A + 2B, and 3A+3B)

5) The number of crimps/cm is calculated

It needs to be kept in mind that the value is calculated from a 2D picture of a 3D object and that the length of the fiber in the z direction is not covered. The real length of the fiber would probably be higher. Also, the 2D picture can mask certain crimps on the fiber, especially in the vicinity of a bonding point. Nevertheless, it is expected that the described estimation can provide a relevant picture of fiber crimping.

The **"length of the filament to the length of the fabric ratio"** can be measured in two different ways:

a) the length of the filaments is measured by stretching them out so that they extend along a line without exhibiting the crimps

b) in a fabric bonded to a given level, it is not possible to use method a) to measure the length of the filaments, so that the following estimation may be used:

a. A picture of the assessed layer is provided in such a magnification that the fibers can be well seen

b. One single fiber is chosen and its path through the picture or at least through part of the picture is marked

c. The length of fiber marked on the picture is measured

d. The length of the fabric, where the fiber is marked is measured

c) The length of the filament to the length of the fabric ratio (percentage) is calculated

The **"resilience"** of a nonwoven is measured according to the European standard test EN ISO 964-1 with following modification:

1.on the sample of a nonwoven is appplied preload force 1,06N.

2.on the sample of a nonwoven is appplied force 5 N with loading speed 5 mm/min.

Resilience is than calculated by following equation:

$$C[\%] = \frac{T_1[mm]}{T_0\ [mm]} \cdot 100\ \%$$

C = resilience [%]

$T_1$ = distance between clamps under the load 5N [mm]

To = thickness (acc. EN ISO 9073-2:1995) [mm]

The **"Bulk density"** of the nonwoven material is calculated by following equation:

$$\rho_b[kg/m^3] = \frac{BW[g/m^2]}{T\ [mm]}$$

$\rho_b$ = bulk density [kg/m$^3$]

BW= basis weight (acc. EN ISO 9073-1:1989) [g/m$^2$]

T = thickness (acc. EN ISO 9073-2:1995) [mm]The bulk density of one layer in a composite:

1) The thickness of a single layer using the optical method from the cross section of a nonwoven is measured. The number of samples shall be at least 10 and the number shall be set so that the corrected sample standard deviation $s$ shall be smaller than 30% of the average value (v is below 30%)

2) The basis weight is measured

a. The production value is taken

b. To obtain an approximate value, it is possible to do the following:

i. A sample of a known surface area is taken

ii. The layers are carefully separated from each other, or the fibers from the layers are separated out,

iii. The weight of the separated layers and the fibers from them are measured.

iv. The basis weight is calculated from the known surface area and the weight of layer.

v. The number of samples shall be at least 10 and the number shall be set so that the corrected sample standard deviation s shall be smaller than 20% of the average value (v is below 20%)

The corrected sample standard deviation shall be calculated using following formula:

$$s = \sqrt{\frac{1}{N-1}\sum_{i=1}^{N}(x_i - \bar{x})^2}.$$

$$v = \frac{s}{\bar{x}} \cdot 100\ (\%)$$

Where:

N - number of samples

xi - single measured value

x - average measured value

**Claims**

1. A spunbond nonwoven web for an acquisition/distribution layer, the web comprising

   - a first layer (1) of filaments, wherein the first layer consists of continuous crimped bi-component filaments of an eccentric core /sheath structure, the filaments having a diameter in the range of 15 to 35 microns and exhibiting at least 3 crimps/cm,
   - a second filament layer (2) arranged in direct contact on the first layer (1),
   **characterized in that**
   the second layer (2) of filaments comprises continuous crimped bi-component filaments of an eccentric core /sheath structure, the filaments having a diameter, which is smaller than the diameter of the filaments in the first layer (1) and which is in the range of 10 to 20 microns, and exhibiting at least 3 crimps/cm.

2. The spunbond nonwoven web of claim 1, wherein at least some of the filaments of the second layer (2) are mutually thermally bonded via their sheath components and at least some of the filaments of the first and the second layer (1, 2) are mutually thermally bonded together via their sheath components .

3. The spunbond nonwoven web of claim 1 or 2, wherein the core of the filaments of the first layer (1) and/or of the second filament layer (2) consists of PET or PLA or PET copolymer and extends over at least 50 % of the filament cross section.

4. The spunbond nonwoven web of any of claims 1-3, wherein the sheath of the filaments of the first layer (1) and/or of the second layer (2) comprises polyethylene homopolymer or polyethylene copolymer or polypropylene copolymer.

5. The spunbond nonwoven web of any of claims 1-4, wherein the first layer (1) has a lower bulk density than the second layer (2), wherein preferably the difference of bulk density is 5 to 80 kg/m$^3$, preferably 40 to 60 kg/m$^3$.

6. The spunbond nonwoven web according to any of the preceding claims, wherein the degree of bonding at the outer surface of the second layer (2) decreases continually through the nonwoven web in the direction towards the outer surface of the first layer (1).

7. The spunbond nonwoven web of any of claims 1-6, wherein the first layer (1) consists of continuous crimped bi-component filaments having a diameter of 20 to 30 microns, and showing 5-15 crimps/cm, and/or the second layer (2) consists of continuous crimped bi-component filaments having a diameter of 15 to 20 microns and showing 5-15 crimps/cm.

8. The spunbond nonwoven web of any of the preceding claims, the second filament layer (2) is shrunken and/or the first filament layer (1) comprises wrinkles (3) at its outer surface, at least 30% of which form with the machine direction (5) an angle (4) which is in the range of 5 and 20°.

9. Absorbent article comprising an acquisition/distribution layer consisting of the spunbond nonwoven web according to any of the preceding claims, **characterised in that** it further comprises a liquid pervious topsheet, a liquid impervious backsheet joined to said topsheet; an absorbent core positioned between said topsheet and said backsheet, wherein the acquisition/distribution layer is positioned between said topsheet and said absorbent core.

10. The absorbent article according to claim 9, **characterised in that** the acquisition/distribution layer has a length and a width, the filaments extending substantially along the length direction and having a length which is at least 120%, preferably at least 150% most preferably at least 180% the length of the length of the acquisition/distribution layer.

**Patentansprüche**

1. Ein Spinnvlies für eine Aufnahme- und Verteilungsschicht, umfassend

   - eine erste Schicht (1) von Filamenten, wobei die erste Schicht aus kontinuierlichen gekräuselten Bikomponentenfilamenten mit exzentrischer Kern-Mantel-Struktur, wobei die Filamenten einen im Bereich von 15 bis 35 Mikrometer liegenden Durchmesser sowie mindestens 3 Kräuselbogen pro cm aufweisen,
   - eine zweite Schicht (2) von Filamenten, die mit einer direkten gegenseitigen Berührung auf der ersten Schicht (1) angeordnet ist,
   **dadurch gekennzeichnet, dass**
   die zweite Schicht (2) von Filamenten kontinuierliche gekräuselte Bikomponentenfilamenten mit exzentrischer Kern-Mantel-Struktur umfasst, wobei die Filamenten einen Durchmesser, der kleiner ist als der Durchmesser der in der ersten Schicht (1) enthaltenen Filamenten und der im Bereich von 10 bis 20 Mikrometer liegt, sowie mindestens 3 Kräuselbogen pro cm aufweisen.

2. Das Spinnvlies nach Anspruch 1, wobei zumindest einige der die zweite Schicht (2) bildenden Filamenten miteinander mittels deren Mantelkomponenten thermisch gebunden sind und zumindest einige der die erste sowie zweite Schicht (1, 2) bildenden Filamenten miteinander mittels deren Mantelkomponenten thermisch gebunden sind.

3. Das Spinnvlies nach Anspruch 1 oder 2, wobei der Kern der die erste Schicht (1) und/oder die zweite Schicht (2) bildenden Filamenten aus PET oder PLA oder einem PET-Kopolymer besteht und sich über mindestens 50 % des Querschnitts der Filamenten erstreckt.

4. Das Spinnvlies nach einem der Ansprüche 1 bis 3, wobei der Mantel der die erste Schicht (1) und/oder die zweite Schicht (2) bildenden Filamenten ein Polyethylen-Homopolymer oder ein Polyethylen-Kopolymer oder ein Polypropylen-Homopolymer umfasst.

5. Das Spinnvlies nach einem der Ansprüche 1 bis 4, wobei die erste Schicht (1) eine niedrigere Schüttdichte als die zweite Schicht (2) aufweist, wobei der Unterschied zwischen den Schüttdichten vorzugsweise 5 bis 80 kg/m$^3$, vorzugsweise 40 bis 60 kg/m$^3$ beträgt.

6. Das Spinnvlies nach einem der vorhergehenden Ansprüche, wobei der auf der äusseren Oberfläche der zweiten Schicht (2) bestehende Bindungsgrad kontinuierlich durch das Vlies hindurch und in Richtung der äusseren Oberfläche der ersten Schicht (1) abnimmt.

7. Das Spinnvlies nach einem der Ansprüche 1 bis 6, wobei die erste Schicht (1) aus kontinuierlichen gekräuselten Bikomponentenfilamenten mit einem im Bereich von 20 bis 30 Mikrometer liegenden Durchmesser und mit 5-15 Kräuselbogen pro cm besteht und/oder die zweite Schicht (2) aus kontinuierlichen gekräuselten Bikomponentenfilamenten mit einem im Bereich von 15 bis 20 Mikrometer liegenden Durchmesser und mit 5-15 Kräuselbogen pro cm besteht.

8. Das Spinnvlies nach einem der vorhergehenden Ansprüche, wobei die zweite Schicht (2) von Filamenten geschrumpft ist und/oder die erste Schicht (1) von Filamenten auf ihrer äusseren Oberfläche Falten (3) aufweist, von denen mindestens 30 % mit der Maschinenrichtung (5) einen Winkel (4) einschliesst, dessen Grösse im Bereich von 5 bis 20° liegt.

9. Ein saugfähiger Artikel, umfassend eine aus dem Spinnvlies nach einem der vorhergehenden Ansprüche bestehende Aufnahme- und Verteilungsschicht, **dadurch gekennzeichnet, dass** er ferner eine flüssigkeitsdurchlässige Deckschicht, eine mit der besagten Deckschicht verbundene flüssigkeitsundurchlässige Tragschicht sowie einen saugfähigen Kern, der zwischen der besagten Deckschicht und der besagten Tragschicht angeordnet ist, wobei die Aufnahme- und Verteilungsschicht zwischen der besagten Deckschicht und dem besagten saugfähigen Kern angeordnet ist.

10. Der saugfähige Artikel nach Anspruch 9, **dadurch gekennzeichnet, dass** die Aufnahme- und Verteilungsschicht eine Länge sowie eine Breite aufweist, wobei die Filamenten im Wesentlichen entlang der Längsrichtung verlaufen und eine Länge aufweisen, die mindestens 120 %, vorzugsweise mindestens 150 %, vorzüglicherweise mindestens 180 % der Gesamtlänge der Aufnahme- und Verteilungsschicht beträgt.

## Revendications

1. Une bande non-tissée filée-liée pour une couche d'acquisition / distribution, la bande comprenante

    - une première couche (1) de filaments, où la première couche consiste en filaments continus frisés à deux composants avec une structure d'âme-gaine excentrique, les filaments ayants un diamètre dans la plage de 15 à 35 microns et présentants au moins 3 frisures/cm
    - une seconde couche (2) de filaments disposée en contact direct sur la première couche (1), **caractérisée en ce que** la seconde couche (2) de filaments comprend des filaments continus frisés à deux composants avec une structure d'âme-gaine excentrique, les filaments ayants un diamètre, qui est inférieur au diamètre des filaments de la première couche (1) et qui est dans la plage de 10 à 20 microns, et présentants au moins 3 frisures/cm.

2. La bande non-tissée filée-liée selon la revendication 1, où au moins certains des filaments de la seconde couche (2) sont thermiquement reliés mutuellement via leur composants de gaine et au moins certains des filaments de la première et de la seconde couche (1, 2) sont thermiquement reliés mutuellement via leur composants de gaine.

3. La bande non-tissée filée-liée selon la revendication 1 ou 2, où l'âme des filaments de la première couche (1) et/ou de la seconde couche (2) de filaments consiste en PET ou PLA ou copolymère PET et qui s'étend sur au moins 50 % de la section transversale du filament.

4. La bande non-tissée filée-liée selon l'une quelconque des revendications 1-3, où la gaine des filaments de la première couche (1) et/ou la seconde couche (2) comprend un polyéthylène homopolymère ou un polyéthylène copolymère ou un polypropylène copolymère.

5. La bande non-tissée filée-liée selon l'une quelconque des revendications 1-4, où la première couche (1) a une densité apparente inférieure à la seconde couche (2), où de préférence la différence de la densité apparente est de 5 à 80 kg/m$^3$, de préférence de 40 à 60 kg/m$^3$.

6. La bande non tissée filée-liée selon l'une quelconque des revendications précédentes, où le degré de liaison sur la surface extérieure de la seconde couche (2) décroît continuellement à travers la bande dans la direction vers la surface extérieure de la première couche (1).

7. La bande non tissée filée-liée selon l'une quelconque des revendications 1-6, où la première couche (1) consiste en filaments continus frisés à deux composants, ayants un diamètre de 20 à 30 microns et présentants 5-15 frisures/cm, et/ou la seconde couche (2) consiste en filaments continus frisés à deux composants, ayants un diamètre de 15 à 20 microns et présentants 5-15 frisures/cm.

8. La bande non-tissée filée-liée selon l'une quelconque des revendications précédentes, la seconde couche (2) de filaments est rétrécie et/ou la première couche (1) de filaments comprend des plis (3) sur sa surface extérieure, dont au moins 30 % forment au sens machine (5) un angle (4) qui est dans la plage de 5 à 20°.

9. Un article absorbant, comprenant une couche d'acquisition / distribution qui consiste en bande non-tissée filée-liée selon l'une quelconque des revendications précédentes, **caractérisé en ce qu'**il comprend également une feuille supérieure perméable aux liquides, une feuille arrière imperméable aux liquides reliée à ladite feuille supérieure; une âme absorbante positionnée entre ladite feuille supérieure et ladite feuille arrière, où la couche d'acquisition / distribution est positionnée entre ladite feuille supérieure et ladite âme absorbante.

10. L'article absorbant selon la revendication 9, **caractérisé en ce que** la couche d'acquisition / distribution a une longueur et une largeur, les filaments s'étendants essentiellement dans le sens longitudinal et ayants une longueur qui est au moins 120%, de préférence au moins 150%, idéalement au moins 180% la longueur de la longueur de la couche d'acquisition / distribution.

Fig. 1

Fig. 2

Fig. 3-1

Fig. 3-2

Fig. 3-3

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20150148764 A, Latimer

- WO 2012130414 A